# EUROPÄISCHE PATENTSCHRIFT

(11) **EP 1 904 994 B1**
(45) Veröffentlichungstag und Bekanntmachung des Hinweises auf die Patenterteilung: **11.01.2012**
(21) Anmeldenummer: 06753724.1
(22) Anmeldetag: 18.05.2006
(51) Int. Cl.: G09F 19/20

(54) **VERFAHREN ZUM MISCHEN VON FARBEN IN EINEM DISPLAY**
METHOD FOR MIXING COLORS IN A DISPLAY UNIT
PROCEDE DE MELANGE DE COULEURS DANS UN DISPOSITIF D'AFFICHAGE

(30) Priorität: 21.05.2005 DE 102005023617
(43) Veröffentlichungstag der Anmeldung: 02.04.2008
(73) Patentinhaber: ASPRE AG, 9050 Appenzell (CH)
(72) Erfinder: SACHER, Friedrich-Josef, 53842 Troisdorf (DE)
(74) Vertreter: Patentanwälte Freischem
(86) Internationale Anmeldenummer: PCT/EP2006/004742
(87) Internationale Veröffentlichungsnummer: WO 2006/125563

(56) Entgegenhaltungen:
- EP-B1- 1 090 384
- WO-A-2004/068208
- WO-A-2004/079439
- DE-A1- 10 237 069

## Beschreibung

Die Erfindung bezieht sich auf ein Verfahren zum Mischen von Farben in einem Display mit rasterartig angeordneten Pixeln, bei dem jeder Pixel mindestens drei hintereinander oder nebeneinander angeordnete Farbspiegel aufweist, die von flachen durchsichtigen Behältern gebildet sind, deren Innenräume über Kanäle mit Reservoirs in Verbindung stehen und in die Farbspiegel und deren Reservoirs eine Farbflüssigkeit und ein klar durchsichtiges, farbloses Medium, das nicht mit der Farbflüssigkeit mischbar ist, bewegt werden kann. Diese Displays können mit Auflicht und auch mit Hinterleuchtung arbeiten.

Sind die Farbspiegel eines Pixels hintereinander angeordnet, dann müssen die Behälter aus klar durchsichtigem Material bestehen, und die Farbflüssigkeit muß transparent sein. Ferner muß sich hinter den Farbspiegeln eine weiße oder silbrig-weiße Reflexionsschicht befinden, falls es sich um ein Auflicht-Display handelt.

Sind bei einem Auflicht-Display die Farbspiegel eines Pixels nebeneinander angeordnet, darin kann der Boden der Farbspiegel weiß oder schwarz sein und - sofern die Farbspiegel aus klar durchsichtigem Material bestehen - kann hinter den Farbspiegeln eine weiße oder schwarze Fläche angeordnet sein, so daß der Farbspiegel weiß oder schwarz erscheint, wenn er mit dem farblosen, klar durchsichtigen Medium gefüllt ist.

Die Pixel und deren Farbspiegel sind bei quadratischer Ausbildung kleiner als 3 mm Kantenlänge und haben ein Volumen von kleiner als 0,5 mm³. Die Förderung der Farbflüssigkeit in die Farbspiegel kann über Mikropumpen, Piezoaktoren, Electrowetting oder Druckgas erfolgen. Sind die Farbspiegel eines Pixels hintereinander angeordnet, dann erfolgt das Mischen der Farben mittels der drei Sekundärfarben Cyan, Magenta und Gelb subtraktiv. Diese Farben wirken als Filter, wobei Cyan aus dem weißen Licht die Primärfarbe Rot und Magenta die Primärfarbe Grün und Gelb die Primärfarbe Blau ausfiltert. Werden die drei Filter Cyan, Magenta und Gelb übereinandergelegt, dann werden die drei Primärfarben Rot, Grün und Blau und damit das Licht ausgefiltert, so daß das Pixel schwarz erscheint.

Das Mischen von Farbe auf subtraktive und additive Weise sowie Displays der eingangs genannten Art sind bekannt aus den Patentschriften EP 1 090 384, US 6 037 955 und US 6 747 777.

Obgleich durch Mischen der Primärfarben oder der Sekundärfarben theoretisch alle Farben erzeugt werden können, ist es in der Praxis steuerungstechnisch und optisch sehr schwierig, den optimalen Farbton und die richtige Farbintensität bei oft wechselndem Auflicht in den relativ kleinen Farbspiegeln und Pixeln zu erzeugen.

Der Erfindung liegt die Aufgabe zugrunde, ein Verfahren bereitzustellen, mit dem das Mischen von Farben erleichtert ist.

Bei einem Display der eingangs genannten Art kann erfindungsgemäß das Mischen der Farben in der Weise erfolgen, daß die Farbflüssigkeit und das klar durchsichtige Medium in schneller Folge abwechseln in die Farbspiegel bewegt werden, wobei der zeitliche Anteil, in dem die Farbflüssigkeit oder das klar durchsichtige Medium sich im Farbspiegel befindet, steuerbar ist.

Die Geschwindigkeit, mit welcher der Wechsel von Farbflüssigkeit und klar durchsichtigem farblosen Medium im Farbspiegel zu erfolgen hat, ist abhängig von der Trägheit des menschlichen Auges, die einzelnen Farbwechsel wahrzunehmen. Eine Frequenz von 10 Farbwechseln pro Sekunde kann ausreichend sein. Der Farbwechsel ist aber auch mit größerer Frequenz ohne weiteres möglich. Wird beispielsweise in einem Auflicht-Display, dessen Pixel sich aus drei hintereinander angeordneten Farbspiegeln zusammensetzen, hinter denen eine weiße Reflexionsschicht angeordnet ist, zwei Farbspiegel mit farblosem, klar durchsichtigem Medium gefüllt, dann bestimmt nur die in den dritten Farbspiegel eingefüllte Farbflüssigkeit die Farbe des Pixels. Diese Farbe kann dadurch aufgehellt werden, daß in schneller Folge die Farbflüssigkeit und das farblose klar durchsichtige Medium in den Farbspiegel transportiert werden. Je größer der zeitliche Anteil ist, in dem sich das klar durchsichtige Medium im Farbspiegel befindet, um so heller ist die Farbe des Pixels.

Der schnelle Wechsel von Farbflüssigkeit und farbloser, klar durchsichtiger Flüssigkeit ist nur innerhalb eines Farbspiegels möglich. Innerhalb der drei Farbspiegel eines Pixels können aber auch die Farben Rot, Grün, Blau oder Cyan, Magenta und Gelb durch schnelle Wechsel der Farbflüssigkeiten gemischt werden. Dabei wird der zeitliche Anteil gesteuert, in dem die Farbflüssigkeiten Cyan oder Magenta oder Gelb sich in ihren Farbspiegeln. befinden.

Je größer der Zeitanteil einer Farbflüssigkeit ist, um so bestimmender ist diese Farbflüssigkeit in der wahrnehmbaren Mischfarbe des Pixels.

Der Farbwechsel kann beispielsweise - wie beim Kinofilm der Bildwechsel - mit einer Frequenz von 24 bis 30 Hz erfolgen. Falls erforderlich, kann die Frequenz auch erhöht werden. Wenn 33,3% der Zeit innerhalb einer Sekunde die blaue Farbflüssigkeit und 33,3% der Zeit die gelbe Farbflüssigkeit und 33,3% der Zeit eine klar durchsichtige und farblose Flüssigkeit in die Farbspiegel bewegt werden, dann erscheint das Pixel hellgrün. Wird der blaue Zeitanteil auf Kosten der Zeitanteile von gelb und farblos vergrößert, dann tendiert die Farbe des Pixels zu dunkelgrün hin. Neben der Mischung von zwei Farbflüssigkeiten und einer klar durchsichtigen Flüssigkeit ist auch die zeitabhängige Mischung von drei Farben beziehungsweise drei Farbflüssigkeiten Rot, Grün Blau oder Cyan, Magenta, Yellow möglich.

## Patentansprüche

1. Verfahren zum Mischen von Farben in einem Display mit rasterartig angeordneten Pixeln, bei dem jeder Pixel mindestens drei hintereinander oder nebeneinander angeordnete Farbspiegel aufweist, die von durchsichtigen Behältern gebildet sind, deren Innenräume über Kanäle mit Reservoirs in Verbindung stehen, und bei dem in die Farbspiegel und deren Reservoirs eine Farbflüssigkeit und ein mit der Farbflüssigkeit nicht mischbares, klar durchsichtiges Medium bewegt werden kann, **dadurch gekennzeichnet, dass** die Farbflüssigkeit und das klar durchsichtige Medium in schneller Folge von mindestens 10 Wechseln pro Sekunde abwechselnd in die Farbspiegel bewegt werden, wobei der zeitliche Anteil, in dem die Farbflüssigkeit oder das klar durchsichtige Medium sich im Farbspiegel befindet, steuerbar ist.

2. Verfahren zum Mischen von Farben in einem Display mit rasterartig angeordneten Pixeln, bei dem jeder Pixel mindestens drei hintereinander oder nebeneinander angeordnete Farbspiegel aufweist, die von durchsichtigen Behältern gebildet sind, deren Innenräume über Kanäle mit Reservoirs in Verbindung stehen, und bei dem in die Farbspiegel und deren Reservoirs eine Farbflüssigkeit und ein mit der Farbflüssigkeit nicht mischbares, klar durchsichtiges Medium bewegt werden kann, **dadurch gekennzeichnet, dass** die Farbflüssigkeiten der drei Farbspiegel eines Pixels in schneller Folge von mindestens 10 Wechseln pro Sekunde abwechselnd in die Farbspiegel bewegt werden, wobei der zeitliche Anteil, in dem eine Farbflüssigkeit eines Farbspiegels oder die Farbflüssigkeit eines anderen Farbspiegels sich im jeweiligen Farbspiegel befindet, steuerbar ist.

## Claims

1. A method for mixing colours in a display unit having a raster-like arrangement of pixels in which each pixel has at least three colour levels arranged one behind the other or side by side, formed by transparent chambers whose interiors are connected to reservoirs via channels, a coloured liquid plus a clearly transparent medium not miscible with the coloured liquid being movable into the colour levels and their reservoirs, **characterized in that** the coloured liquid and the clearly transparent medium are alternately moved in rapid succession of at least 10 alternations per second into the colour levels, the time portion during which the coloured liquid or the clearly transparent medium is located in the colour level being controllable.

2. A method for mixing colours in a display unit having a raster-like arrangement of pixels in which each pixel has at least three colour levels arranged one behind the other or side by side, formed by transparent chambers whose interiors are connected to reservoirs via channels, a coloured liquid plus a clearly transparent medium not miscible with the coloured liquid being movable into the colour levels and their reservoirs, **characterized in that** the coloured liquids of the three colour levels of a pixel are alternately moved in rapid succession of at least 10 alternations per second into the colour levels, the time portion during which the coloured liquid of a colour levels or the coloured liquid of another colour level is located in the respective colour level being controllable.

## Revendications

1. Procédé de mélange de couleurs dans un affichage comprenant des pixels agencés en matrice, dans lequel chaque pixel présente au moins trois zones de coloration agencées les unes derrière les autres ou les unes à côté des autres, qui sont formées par des réceptacles transparents dont les espaces intérieurs sont reliés à des réservoirs par des canaux, et dans lequel un fluide coloré et un milieu transparent non miscible avec le fluide coloré peuvent être déplacés dans les zones de coloration et leurs réservoirs, **caractérisé en ce que** le fluide coloré et le milieu transparent sont déplacés alternativement dans les zones de coloration à un rythme élevé d'au moins 10 changements par seconde, la fraction de temps pendant laquelle le fluide coloré ou le milieu transparent se trouve dans la zone de coloration pouvant être commandée.

2. Procédé de mélange de couleurs dans un affichage comprenant des pixels agencés en matrice, dans lequel chaque pixel présente au moins trois zones de coloration agencées les unes derrière les autres ou les unes à côté des autres, qui sont formées par des réceptacles transparents dont les espaces intérieurs sont reliés à des réservoirs par des canaux, et dans lequel un fluide coloré et un milieu transparent non miscible avec le fluide coloré peuvent être déplacés dans les zones de coloration et leurs réservoirs, **caractérisé en ce que** les fluides colorés des trois zones de coloration d'un pixel sont déplacés alternativement dans les zones de coloration à un rythme élevé d'au moins 10 changements par seconde, la fraction de temps pendant laquelle un fluide coloré d'une zone de coloration ou le fluide coloré d'une autre zone de coloration se trouve dans la zone de coloration correspondante pouvant être commandée.
